# NEUE EUROPÄISCHE PATENTSCHRIFT

(11) **EP 2 522 983 B2**
(45) Veröffentlichungstag und Bekanntmachung des Hinweises auf die Entscheidung über den Einspruch: **30.03.2022**
(45) Hinweis auf die Patenterteilung: 16.12.2015
(21) Anmeldenummer: 12167890.8
(22) Anmeldetag: 14.05.2012
(51) Int. Cl.: G01N 15/14

(54) **Düse zur Partikelausrichtung im Flüssigkeitsstrom**
Nozzle for particle alignment in fluid flows
Buse pour orientation des particules dans un flux de liquide

(30) Priorität: 12.05.2011 DE 102011075711
(43) Veröffentlichungstag der Anmeldung: 14.11.2012
(73) Patentinhaber: Masterrind GmbH, 27283 Verden (DE)
(72) Erfinder: Rath, Detlef, 31535 Neustadt (DE)
(74) Vertreter: Taruttis, Stefan Georg

(56) Entgegenhaltungen:
- EP-A2- 0 288 029
- WO-A1-94/08223
- WO-A1-2009/151624
- US-A1- 2004 050 186
- US-A1- 2004 095 574
- KACHEL V ET AL: "UNIFORM LATERAL ORIENTATION, CAUSED BY FLOW FORCES, OF FLAT PARTICLES IN FLOW-THROUGH SYSTEMS", JOURNAL OF HISTOCHEMISTRY AND CYTOCHEMISTRY, HISTOCHEMICAL SOCIETY, NEW YORK, NY, US, Bd. 25, Nr. 7, 1. Januar 1977 (1977-01-01) , Seiten 774-780, XP002915897, ISSN: 0022-1554

## Beschreibung

Die vorliegende Erfindung betrifft eine Düse zur Ausrichtung von Partikeln in einem Flüssigkeitsstrom, der eine partikelhaltige Kernstromflüssigkeit innerhalb einer Hüllstromflüssigkeit aufweist oder daraus besteht, wobei die Düse ein Zuführungsröhrchen für Kernstromflüssigkeit aufweist, ein Durchflusszytometer mit der Düse und ein Verfahren zur Ausrichtung von Partikeln in einem Flüssigkeitsstrom, bevorzugt ein Verfahren zur Herstellung zumindest einer Fraktion der Partikel mit dem Schritt des Ausrichtens der Partikel und des Auftrennens der Partikel in die zumindest eine Fraktion und eine weitere Fraktion. Bevorzugte Partikel, die in einem Flüssigkeitsstrom mit der Düse ausgerichtet werden können, sind von insgesamt flacher Gestalt, und sind beispielsweise zu einer Schnittebene symmetrisch, sodass die Partikel senkrecht zu ihrer Längsachse einen Querschnitt mit einer ersten und einer zweiten Dimension aufweisen, wobei der Querschnitt in der ersten Dimension kleiner ist als in der zweiten Dimension. Bevorzugte Partikel sind biologische Zellen, beispielsweise Blutzellen, insbesondere plättchenförmige Zellen, menschliche und nicht-menschliche Säugetierspermien, insbesondere von einem männlichen Rind, Schwein, Schaf, Elefanten, Kamel oder Pferd gewonnene Spermien.

### Stand der Technik

Die US 6,149,867 beschreibt eine gattungsgemäße Düse und ein Verfahren mit einer Düse zur Sortierung von Säugerspermien in geschlechtschromosomenspezifische Spermienfraktionen. Das Verfahren verwendet ein Durchflusszytometer mit einer konisch zulaufenden Düse, die in ihrem Volumen eine Zuleitung für die Spermien enthaltende Kernflüssigkeit enthält, sodass am Düsenauslass ein von einem Hüllstrom umgebener Spermien enthaltender Kernstrom austritt. Die Geometrie der Zuleitung für Spermien und insbesondere die Auslassöffnung des Zuführröhrchens werden nicht näher beschrieben.

Die WO 99/05504 beschreibt eine Düse zur Verwendung in einem Durchflusszytometer zur Sortierung von Spermien, die sich dadurch auszeichnet, dass die Düse in dem Abschnitt zwischen der Auslassöffnung eines Zuführröhrchens für Spermien enthaltende Kernstromflüssigkeit eine im Wesentlichen trichterförmig zulaufende Innenoberfläche hat, die einen ersten ellipsenförmigen Querschnitt hat, und einen daran angrenzenden axialen Abschnitt mit ellipsenförmigem Querschnitt, dessen Längsachse um 90° gedreht ist. Das Zuführröhrchen hat eine zylindrische Form und kann im Bereich der Auslassöffnung abgeschrägt sein.

Die EP 1238261 B1, beschreibt eine Düse für ein Durchflusszytometer, bei dem ein in einer Düse koaxial angeordnetes Zuführröhrchen für Spermien enthaltende Kernstromflüssigkeit von einem Abschnitt mit zylindrischem Außendurchmesser zu einem im Wesentlichen rechteckigen Querschnitt innerhalb eines kegelstumpfförmigen Abschnitts der Düse zuläuft, wobei sich stromabwärts dieses Zulaufröhrchens ein sich verjüngender Abschnitt der Düse mit elliptischem Querschnitt anschließt. Der Auslass des Zuführröhrchens ist abgeschrägt und hat im Querschnitt eine im Wesentlichen runde Öffnung.

Die US 2004/0095574 A1 beschreibt eine Durchflusszelle mit einer Kanüle zur Injektion einer Probenflüssigkeit in einem Flüssigkeitskanal für Hüllflüssigkeit, dessen Querschnitt sich bis zu einem flachen Querschnitt eines Beobachtungsabschnitts verjüngt, auf den ein Mikroskop gerichtet ist. Die Kanüle läuft von einem runden Querschnitt zu einem elliptischen Querschnitt an ihrem Auslass zu, der eine größere Breite als der Beobachtungsbereich des Mikroskops aufweist, z.B. 2mm, und eine Höhe von z.B. 50-400µm. Die Fokussierung der Probenflüssigkeit in eine Ebene erfolgt zum einen durch die Verjüngung des Flüssigkeitskanals für die Hüllflüssigkeit, die stromabwärts der Kanüle angeordnet ist und zum anderen durch unterschiedliche Strömungsgeschwindigkeiten von Probenflüssigkeit und Hüllflüssigkeit.

Die WO 2009/151624 A1 beschreibt eine Düse zur Ausrichtung von Partikeln, bei der ein innenliegendes Röhrchen für Probenflüssigkeit abschnittsweise einen runden Außenquerschnitt aufweisen kann, der zu einem rechteckigen Außenquerschnitt am Auslassende übergeht. Der Innenquerschnitt dieses Röhrchens ist immer zylindrisch.

Die US 2004/0050186 A1 beschreibt eine Düse, die sich durch eine an den Düsenauslass angrenzende zulaufende Innenfläche mit elliptischem Querschnitt auszeichnet. Das in der Düse angeordnete Zuführungsröhrchen kann einen rechteckigen Außenquerschnitt haben.

Die EP 0288029 A2 beschreibt eine Mikrofluidikkammer zur optischen Analyse von Zellen, die einen Strömungskanal für Hüllflüssigkeit mit einem sich zu einem Rechteckquerschnitt verjüngenden Auslass aufweist, wobei in dem Strömungskanal eine Düse für Probenflüssigkeit liegt.

Kachel et al., The Journal of Histochemistry and Cytometry 774-780 (1977) beschreiben die Ausrichtung von Partikeln in einer Düse, die stromabwärts des runden Zuführungsröhrchens für partikelhaltige Kernstromflüssigkeit einen sich verjüngenden gestreckten Querschnitt zur hydrodynamischen Ausrichtung der Partikel aufweist.

Diese bekannten Düsen für Durchflusszytometer erzeugen durch den einfach oder doppelt elliptischen Innenquerschnitt der Düse eine Ausrichtung der Partikel. Dies wird auf eine abschnittsweise Druckerhöhung durch die zwei gegenüberliegenden Düsenflächen zurückgeführt, die im kleineren Ellipsenradius näher aneinander liegen als die Düsenflächen, die im größeren Ellipsenradius liegen. Da diese asymmetrischen Druck- und Strömungsverhältnisse auf den Hüllstrom und den darin angeordneten Kernstrom wirken, wird dieses Verfahren auch als hydrodynamische Ausrichtung der Partikel bezeichnet.

### Aufgabe der Erfindung

Gegenüber dem Stand der Technik liegt der vorliegenden Erfindung die Aufgabe zugrunde, eine alternative Düse zur Partiketausrichtung und ein Verfahren zur Ausrichtung nicht rotationssymmetrischer Partikel, insbesondere Säugerspermien, in einem Flüssigkeitsstrom bereitzustellen.

### Allgemeine Beschreibung der Erfindung

Die Erfindung löst die Aufgabe durch eine Düse gemäss Anspruch 1 und ein Verfahren gemäss Anspruch 14.

Der an die Mündung des Zuführungsröhrchens angrenzende Abschnitt erstreckt sich über die vollständige Länge des Zuführungsröhrchens, die innerhalb der Düse angeordnet ist, insbesondere über den Abschnitt des Zuführungsröhrchens zwischen dem ersten Ende der Düse, das gegenüber ihrer Auslassöffnung liegt, und seiner Mündung. Das Zuführungsröhrchen endet in einer Mündung, die in einem Abstand zur Auslassöffnung der Düse angeordnet ist, so dass aus der Mündung des Zuführungsröhrchens ausströmende Kernstromflüssigkeit von Hüllstromflüssigkeit umgeben wird, durch den zu Auslassöffnung zulaufenden Innenquerschnitt beschleunigt und gestreckt wird und aus der Auslassöffnung austritt.

Erfindungsgemäss wird die Ausrichtung der Partikel durch den gemäss Anspruch 1 bzw. Anspruch 14 gestreckten Innenquerschnitt des Zuführungsröhrchens bewirkt. In dem sich an die Mündung des Zuführungsröhrchens anschliessenden Abschnitt der Düse bis zu deren Auslassöffnung wird die aus dem Zuführungsröhrchen austretende Kemstromflüssigkeit mit den darin enthaltenen Partikeln von Hüllstromflüssigkeit kontaktiert. Da die Partikel auf Grund ihres Durchtritts durch das Zuführungsröhrchen eine bezüglich des Innenquerschnitts des Zuführungsröhrchens bzw. eine bezüglich ihrer flachen Gestalt konstante Ausrichtung aufweisen, erfolgt bei Verwendung der Düse durch eine gegenüber der Kernstromflüssigkeit höhere Geschwindigkeit der Hüllstromflüssigkeit eine Beabstandung der Partikel voneinander, wobei eine hydrodynamische Ausrichtung durch einen seitlichen Druckgradienten auf die Kemstromflüssigkeit, der durch eine elliptische Düse erzeugt wird, nur zusätzlich zur Ausrichtung beiträgt. Bevorzugt ist die Düse eingerichtet, dass ein seitlich auf die Kernstromflüssigkeit wirkender Druckgradient nicht auftritt, z.B. durch eine rotationssymmetrische Form des Abschnitts der Düse zumindest zwischen der Mündung des Zuführungsröhrchens und der Auslassöffnung der Düse.

Der Innenquerschnitt des Zuführungsröhrchens der erfindungsgemäßen Düse erlaubt eine Ausrichtung der artikel, z.B. mit deren anschließender Trennung in Fraktionen, vorzugsweise bei hohen Geschwindigkeiten bzw. Sortierraten, da die Beschleunigung der Kernstromflüssigkeit durch eine höhere Geschwindigkeit im Wesentlichen nur zu Beschleunigung längs der Düsenachse führt, ohne die Einheitlichkeit der Ausrichtung der Partikel nach Austritt aus dem Zuführungsröhrchen signifikant zu verändern. Im Unterschied dazu führen bei herkömmlichen Düsen mit rotationssymmetrischem Zuführungsröhrchen und einer einfach oder doppelt elliptischen Düse in dem Düsenabschnitt zwischen Mündung des Zuführungsröhrchens und Auslassöffnung der Düse höhere Geschwindigkeiten der Hüllstromflüssigkeit zur Änderung des seitlichen Druckgradienten auf die Kernstromflüssigkeit und daher zur ausschließlich hydrodynamischen Ausrichtung der Partikel. Daher führt bel herkömmlichen Düsen eine höhere Geschwindigkeit zu einer weniger einheitlichen Ausrichtung der Partikel, während eine gute Ausrichtung bei niedrigen Geschwindigkeiten erreicht wird. Für die Zwecke der Erfindung kann das Zuführungsröhrchen auch ein umfänglich geschlossener Kanal sein und als solcher bezeichnet werden.

Die erfindungsgemäße Düse kann einen einfach oder doppelt elliptischen Innenquerschnitt aufweisen und ist vorzugsweise rotationssymmetrisch, d.h. mit rundem, zulaufendem innenquerschnitt, da die Ausrichtung der Partikel innerhalb des Zuführungsröhrchens erfolgt, das den Kernstrom formt. Da die Ausrichtung der Partikel allein im Kernstrom, d.h. ohne Kontakt mit der Hüllstromflüssigkeit erfolgt, kann das erfindungsgemäße Verfahren auch als mechanische Ausrichtung der Partikel bezeichnet werden. Es wird angenommen, dass sich die Partikel dadurch in dem in einer Dimension gestreckten Querschnitt des Zuführungsröhrchens ausrichten, dass sich beim Durchströmen des Zuführungsröhrchens mit der partikelhaltigen Kernstromflüssigkeit eine laminare Strömung mit einem Geschwindigkeitsgradienten ausbildet, der die Partikel ausrichtet.

Generell betrifft die Erfindung eine Düse nach Anspruch 1 zur Partikelausrichtung für ein Durchflusszytometer mit einem ersten Ende und einem gegenüberliegenden zweiten Ende und einem Im Innenvolumen der Düse angeordneten Zuführungsröhrchen für Kernstromflüssigkeit, das In einem an (seine Mündung angrenzenden Abschnitt koaxial in der Düse angeordnet ist, wobei das Zuführungsröhrchen eine erste Einlassöffnung für Kernstromflüssigkeit aufweist und eine Mündung, die zu einer am zweiten Ende der Düse angeordneten Auslassöffnung beabstandet ist, und die Düse eine zweite Einlassöffnung für Hüllstromflüssigkeit aufweist, wobei der Innenquerschnitt des Zuführungsröhrchens für Kernstromflüssigkeit zumindest in einem an seine Mündung angrenzenden Abschnitt in einer ersten Dimension kleiner ist als in einer zweiten Dimension. Bevorzugt weist das Zuführungsröhrchen einen rechteckigen Innenquerschnitt auf, dessen Längserstreckung in der zweiten Dimension liegt, insbesondere weist das Zuführungsröhrchen einen ovalen Innenquerschnitt auf, dessen Längserstreckung in der zweiten Dimension liegt. Insbesondere ist die erste Dimension um zumindest den Faktor 0,3 bis 0,9 kleiner als die zweite Dimension. Die Düse kann einen elektrischen Anschluß aufweisen, der in das Innenvolumen der Düse ragt. Bevorzugt weist die Düse einen Piezokristall in einem Abstand zum ersten Ende der Düse an einer Wand auf, die den Querschnitt der Düse bis auf das Zuführungsröhrchen verschließt, so dass die Düse zur Erzeugung eines Tröpfchenstroms eingerichtet ist.

Die erfindungsgemäße Düse lässt sich in vier Abschnitte I, II, III und IV einteilen. Abschnitt I umfasst das erste Ende der Düse, welches von einem Halter gebildet wird, und kann im Wesentlichen eine zylindrische, würfelförmige, quaderförmige oder prismatische Form annehmen. Abschnitt II liegt zwischen Abschnitt I und Abschnitt III und weist eine zulaufende Form mit z.B. elliptischem Innenquerschnitt auf oder mit einem rotationssymmetrischen Innenquerschnitt, insbesondere mit rotationssymmetrischer Zylinderform oder Kegelstumpfform. Bevorzugt ist der Innenquerschnitt in Abschnitt II ein sich zur Düsenauslassöffnung verringernder rechteckiger Innenquerschnitt oder ein zur Längsachse des Zuführungsröhrchens punktsymmetrischer Innenquerschnitt, in dessen Innenvolumen das Zuführungsröhrchen für Kernströmflüssigkeit verläuft, bevorzugt koaxial. Der Innenquerschnitt des Abschnitts II kann z.B. durch einen Träger und einen dazu parallelen Deckel begrenzt werden, die vorzugsweise plan sind und das Zuführungsröhrchen umfassen. In dieser Ausführungsform bilden Träger und Deckel zwischen sich, dem Zuführungsröhrchen und Wänden, die von Zuführungsröhrchen beabstandet sind und Träger und Deckel verbinden, den Innenquerschnitt für Hüllflüssigkeit. Das Innenvolumen der Düse in Abschnitt II umfasst in dieser Ausführungsform das Zuführungsröhrchen und wird bei dem erfindungsgemäßen Verfahren im Wesentlichen von Hüllstromflüssigkeit ausgefüllt. Abschnitt III ist zwischen Abschnitt II und Abschnitt IV angeordnet und weist bevorzugt einen zur Längsachse des Zuführungsröhrchens symmetrischen Innenquerschnitt auf, z.B. eine vollständig rotationssymmetrischen Innenquerschnitt oder einen zwischen einem Träger und einem gegenüberliegenden Deckel, die in einem Abstand parallel zur Längsachse des Zuführungsröhrchens angeordnet sind, angeordneten Abschnitt einer rotationssymmetrischen, bevorzugt zylindrischen Form, welche im Wesentlichen von dem dem ersten Ende der Düse zugewandten Abschnitt eines am zweiten Ende der Düse angeordneten Düseneinsatzes gebildet wird. Der Abschnitt III kann z.B. einen vollständig rotationssymmetrischen Innenquerschnitt, z.B. kegelstumpfförmig mit koaxialem Zuführungsröhrchen, oder einen Innenquerschnitt aus zwei durch das Zuführungsröhrchen beabstandeten Abschnitten mit jeweils rechteckigem Querschnitt aufweisen, die einen Abschnitt des vollständig rotationssymmetrischen Innenquerschnitts bilden, wobei sich der Innenquerschnitt in Richtung auf den Abschnitt IV verjüngen kann. Die Abschnitte mit jeweils rechteckigem Querschnitt können zwischen dem Träger und dem Deckel gebildet sein und in einem Abstand beidseitig zum Zuführungsröhrchen durch die Wänden der Düse begrenzt sein. Im Inneren des Abschnitts III der Düse verläuft bevorzugt koaxial zur Düse das Zuführungsröhrchen für Kernstromflüssigkeit, dessen Mündung an dem dem zweiten Ende der Düse zugewandten Ende von Abschnitt III angeordnet ist. Dieser Abschnitt III kann, z.B. zur Ausrichtung von nicht-menschlichen Säugerspermien eine Länge von 1 bis 200 mm, bevorzugt von 5 bis 100 mm oder bis 50 oder bis 20 mm aufweisen.

Abschnitt IV befindet sich am zweiten Ende der Düse, verjüngt sich zur am zweiten Ende der Düse angeordneten Auslassöffnung und ist optional rotationssymmetrisch, bevorzugt kegelförmig bzw. kegelstumpfförmig ausgebildet oder kann einen sich zur Auslassöffnung der Düse verkleinernden rechteckigen Querschnitt aufweisen. Abschnitt IV kann zwischen einem Träger und einem davon beabstandeten Deckel gebildet sein, die jeweils plan sein können, und durch die Wände der Düse begrenzt sein, die sich zwischen Träger und Deckel erstrecken. Generell ist die Auslassöffnung der Düse bevorzugt kreisförmig. Der Abschnitt IV kann einen rechteckigen Querschnitt aufweisen und geht bevorzugt in einen kreisförmigen Querschnitt über, der die Auslassöffnung der Düse bildet.

Optional kann derAbschnitt II und/oder der Abschnitt III entfallen, so dass die Düse aus dem Zuführungsröhrchen besteht, das in einem an seine Mündung angrenzenden Abschnitt koaxial in einer Düse angeordnet ist, die an die Mündung des Zuführungsröhrchens angrenzt, wobei die Düse eine zweite Einlassöffnung für Hüllstromflüssigkeit aufweist, und die Düse zwischen ihrer Auslassöffnung und der Mündung des Zuführungsröhrchens einen Innenquerschnitt umfasst, der sich bevorzugt zur Auslassöffnung verjüngt. In dieser Ausführungsform ist die Düse entlang ihrer Längsachse und/oder entlang der Längsachse des Zuführungsröhrchens auf den Abschnitt zwischen der Auslassöffnung der Düse und die Mündung des Zuführungsröhrchens begrenzt, z.B. durch eine Wandung, die sich angrenzend an die Mündung erstreckt. Eine solche Wandung, die z.B. das erste Ende der Düse bildet, kann sich eben, konvex oder konkav, insbesondere mit parabelförmigem Querschnitt, um das Zuführungsröhrchen erstrecken und z.B. an eine kegelförmige Düsenwand oder an von der Achse des Zuführungsröhrchens beabstandete Träger und Deckel mit zwischen diesen angeordneten Wänden angrenzen, die das Innenvolumen der Düse aufspannen, wobei eine zweite Einlassöffnung für Hüllstromflüssigkeit in der Wandung angeordnet sein kann. Die zweite Einlassöffnung ist insbesondere zum laminaren Einlass von Hüllstromflüssigkeit eingerichtet, z.B. als Elnlassöffnung einer Leitung für Hüllstromflüssigkeit, deren Längsachse parallel zur Längsachse des Zuführungsröhrchens angeordnet Ist, und/oder als Einlassöffnung, die parallel zur Mündung des Zuführungsröhrchens und/oder die in der Ebene der Mündung des Zuführungsröhrchens liegt Die Düse kann insbesondere einen Innenquerschnittaufweisen, wie für jede Ausführungsform für den Abschnitt der Düse zwischen Mündung des Zuführungsröhrchens und Auslassöffnung beschrieben, z.B. in Bezug auf Abschnitt IV.

Die einzelnen Abschnitte können einstückig ausgebildet sein oder miteinander verschweißt oder lösbar miteinander verbunden sein, beispielsweise über Gewinde miteinander verschraubt sein, sodass eine einfache Reinigung der einzelnen Teile durchgeführt werden kann.

Bevorzugt haben die Abschnitte I, II und III eine gemeinsame Längsachse, auf der das Zuführungsröhrchen für Kernstromflüssigkeit verläuft.

Die erfindungsgemäße Düse weist einen Innenquerschnitt entlang einer Längsachse auf, die ein erstes Ende miteinem zweiten Ende verbindet. Der Innenquerschnitt nimmt dabei vom ersten zum zweiten Ende der Düse unabhängig von seiner Form flächenmäßig kontinuierlich ab, wobei er abschnittsweise konstant bleiben kann. Abschnitt I der Düse wird im Wesentlichen von einem Halter gebildet, in welchen eine erste Einlassöffnung für Kernstromflüssigkeit eingelassen Ist, die vom Zuführungsröhrchen für Kernstromflüssigkeit gebildet wird, sodass die in der Kernstromflüssigkeit enthaltenen Partikel direkt in das Zuführungsröhrchen für Kernstromflüssigkeit gepumpt werden können. Das Zuführungsröhrchen verbindet das erste Ende der Düse entlang der Längsachse der Düse mit einem gegenüberliegenden zweiten Ende der Düse.

Das Zuführungsröhrchen für Kernstromflüssigkeit weist senkrecht zu seiner Längsachse eine Aussenquerschnitt und einen Innenquerschnitt auf, welche jeweils in einer ersten und einer zweiten Dimension aufgespannt sind. Die erste Dimension des Innenquerschnitts des Zuführungsröhrchens für die Partikel enthaltende Kernstromflüssigkeit hat dabei ein geringeres Ausmass als in der zweiten Dimension, sodass die Innenquerschnittsfläche des Zuführungsröhrchens gestreckt ist. Dieser entlang der zweiten Dimension gestreckte Innenquerschnitt hat den Vorteil; dass bereits bis zur Mündung des Zuführungsröhrchens eine Ausrichtung der Partikel erreicht wird. Dieser Effekt steigt mit einem betragsmässigen Unterschied von der den Innenquerschnitt des Zuführungsröhrchens aufspannenden ersten und zweiten Dimension, so dass der Innenquerschnitt bevorzugt in der ersten Dimension zumindest um den Faktor 0,3 bis 0,9, bevorzugter um einen Faktor von 0,5 bis 0,8 kleiner als in der zweiten Dimension ist.

Die das Zuführungsröhrchen für Kernstromflüssigkeit passierenden Partikel oder Zellen erfahren durch die Geometrie der inneren Oberfläche des Zuführungsröhrchens Jeweils dieselbe Ausrichtung zum Zuführungsröhrchen, wobei diese Ausrichtung nach Austritt der partikelhaltigen Kernstromflüssigkeit aus der Mündung des Zuführungsröhrchens über den Abschnitt zwischen der Mündung des Zuführungsröhrchens und der Auslassöffnung der Düse erhalten bleibt. Zur besseren Ausrichtung ist bevorzugt, dass der Faktor von erster zu zweiter Dimension des Querschnitts durch die Partikel entlang Ihrer Längsachse gleich dem Faktor von erster zu zweiter Dimension ist, welche den Innenquerschnitt durch das Zuführungsröhrchen für Kernstromflüssigkeit aufspannen.

In einer ersten Ausführungsform ist der Innenquerschnitt des Zuführungsröhrchens elliptisch, in einer zweiten Ausführungsform rechteckig. Ein rechteckiger Innenquerschnitt beträgt in der ersten Dimension 50 µm bis 200 µm, bevorzugt 50 bis 150 µm, und in der zweiten Dimension 75 µm bis 300 µm, bevorzugt bis 200 µm. Bei einem elliptischen Innenquerschnitt beträgt der kleine Durchmesser, der in der ersten Dimension ist, 50 µm bis 200 µm, bevorzugt 50 bis 150 µm und der grosse Durchmesser, der in der zweiten Dimension ist, 75 µm bis 300 µm, bevorzugt bis 200 µm.

Die Wandstärke des Zuführungsröhrchens für Kernstromflüssigkeit kann über die gesamte Länge konstant sein, vom ersten zum zweiten Ende der Düse zunehmen oder vom ersten zum zweiten Ende der Düse abnehmen.

Der Außenquerschnitt des Zuführungsröhrchens für Kernstromflüssigkeit Wird senkrecht zur Längsachse des Zuführungsröhrchens von einer ersten und einer zweiten Dimension aufgespannt, die vorzugsweise parallel zur ersten bzw. zweiten Dimension des Innenquerschnitts aufgespannt sind, wobei der Außenquerschnitt ebenfalls in seiner ersten Dimension kleiner ist als in seiner zweiten Dimension. Die erste und die zweite Dimension, welche den Außenquerschnitt des Zuführungsröhrchens aufspannen, können dabei alternativ unterschiedlich groß sein oder Identische Werte annehmen, sodass der Außenquerschnitt in der Folge unabhängig von der Geometrie des Innenquerschnitts rund oder gestreckt sein kann.

Der senkrecht zur Längsachse aufgespannte Innenquerschnitt bleibt vom ersten Ende der Düse zum zweiten Ende der Düse entlang des Strömungskanals konstant. In einer bevorzugten Aüsfuhrungsform ist der Innenquerschnitt über die gesamte Länge des Zuführungsröhrchens konstant.

Auch der Außenquerschnitt kann sich entlang des Strömungskanals verkleinern oder konstant bleiben. In einer bevorzugten Ausführungsform verjüngt sich der Außenquerschnitt über die Länge des Zuführungsröhrchens. bevorzugt mit über die Länge konstanter Wandstärke.

Das Zuführungsröhrchen für Kernstromflüssigkeit kann optional elektrisch leitend sein, um einen elektrischen Impuls auf die Kernstromflüssigkeit zu übertragen.

Am Halter angeordnet ist eine an eine Hüllflüssigkeitszufuhr angeschlossene zweite Einlassöffnung für Hüllflüssigkeit. Die zweite Einlassöffnung ist bevorzugt in den Halter eingelassen und vorzugsweise näher am ersten Ende der Düse als am zweiten Ende der Düse angeordnet, beispielsweise am Mantel bzw. in einer Wand des Halters. Durch Zuleitung der Hüllflüssigkeit in die Düse ummantelt die Hüllflüssigkeit das Zuführungsröhrchen für Kernstromflüssigkeit, zumindest in Abschnitt IV, der dem zweiten Ende der Düse zugewandt ist. Vorzugsweise verjüngt sich der IV. Abschnitt für Hüllflüssigkeit von dem dem ersten Ende der Düse zugewandten Ende zu seinem dem zweiten Ende der Düse zugewandten Ende kegelstumpfförmig, wobei das Zuführungsröhrchen koaxial darin angeordnet ist.

Der Abschnitt IV der Düse kann von einem Düseneinsatz gebildet sein, der flüssigkeitsdicht und vorzugsweise lösbar an den Abschnitt III anschließt. Sowohl die Hüllflüssigkeitszufuhr als auch das Zuführungsröhrchen münden in den Abschnitt IV bzw. Düseneinsatz, welcher am zweiten Ende der Düse angeordnet und bevorzugt rotationssymmetrisch ist. Der Abschnitt IV, bzw. der Düseneinsatz weist in dem dem zweiten Ende der Düse zugewandten Abschnitt einen sich verjüngenden Abschnitt auf oder besteht daraus. Die Auslassöffnung des Düseneinsatzes, die die Auslassöffnung der Düse bildet, ist bevorzugt kreisförmig gestaltet. Die Auslassöffnung der Düse ist bevorzugt kleiner als die Mündung des Zuführungsröhrchens. Nach einer bevorzugten Ausführungsform ist die Auslassöffnung des Düseneinsatzes senkrecht zu dessen Längsachse im Wesentlichen kreisrund gestaltet.

Das Zuführungsröhrchen für Kernstromflüssigkeit kann an einem Abschnitt der Düse gegenüber ihrer Auslassöffnung befestigt sein, insbesondere am Halter.

In einer bevorzugten Ausführungsform wird das Zuführungsröhrchen von einem Träger, zwei auf dem Träger angeordneten beabstandeten Blöcken, die auch als Stege bezeichnet werden können, und einem zum Träger beabstandeten Deckel gebildet. Bevorzugt sind Träger und Deckel jeweils etwa plan und parallel. Die Blöcke können einstückig mitdem Träger ausgebildet sein. Zwischen Träger und Deckel sind in einem Abstand zu den Blöcken Wände angeordnet, die die Wandung der Düse bilden. Dabei erstrecken sich Träger und Deckel beidseitig des Zuführungsröhrchens bis an die Wände und bilden zwischen sich und den Blöcken und Wänden den Innenquerschnitt der Düse, der sich um das Zuführungsröhrchen erstreckt, z.B. in Abschnitt III und/oder Abschnitt II der Düse. Der Abschnitt IV der Düse zwischen der Mündung des Zuführungsröhrchens und der Auslassöffnung der Düse kann von zwischen Träger und Deckel angeordneten Wänden ausgebildet sein oder von einem Düseneinsatz ausgebildet sein, der flüssigkeitsdicht an die Wände anschließt und sich mit elliptischem oder kreisförmigem Innenquerschnitt zur Auslassöffnung verjüngt. Deckel Und Träger können in dem Abschnitt, in dem sie das Zuführungsröhrchen zwischen den Blöcken begrenzen, im gleichen, einem größeren oder kleineren Abstand angeordnet sein als in dem Abschnitt, in dem sie sich zwischen den Blöcken und den Wänden erstrecken und den Innenquerschnitt der Düse begrenzen. Die Wände in dem Abschnitt der Düse zwischen der Mündung des Zuführungsröhrchens und der Auslassöffnung der Düse können auf dem Träger und/oder dem Deckel angebracht sein, insbesondere einstückig mit dem Träger und/oder dem Deckel ausgebildet sein, und optional erstrecken sich der Träger und der Deckel bis zur Auslassöffnung.

In jeder Ausführungsform kann der Innenquerschnitt des Abschnitts II der Düse zwischen dem Zuführungsröhrchen und ihrer davon beabstandeten Innenwandung entlang der Längsachse der Düse konstant sein oder sich in Richtung auf die Düsenauslassöffnung verjüngen. Bevorzugt weisen die Blöcke den Wandungen der Düse zugewandte Außenflächen auf, die sich in Richtung auf die Mündung des Zuführungsröhrchens verjüngen, bevorzugt Außenflächen, die in dem Abschnitt III, in dem die Wandungen der Düse einen sich zur Auslassöffnung verjüngenden Querschnitt bilden, etwa parallel zu den Wandungen der Düse angeordnet sind.

In dieser Ausführungsform kann die Düse einen Träger und einen vorzugsweise parallelen Deckel, zwischen diesen angeordnete Wände, die den Innenquerschnitt der Düse begrenzen, und zwischen Träger und Deckel angeordnete beabstandete Blöcke aufweisen oder daraus bestehen, wobei optional der Abschnitt der Düse zwischen der Mündung des Zuführungsröhrchens und der Auslassöffnung der Düse von den Wänden oder einem an die Wände, den Träger und den Deckel flüssigkeitsdicht anschließenden Düseneinsatz ausgebildet ist.

Die Herstellung einer Düse dieser Ausführungsform kann durch Anordnen bzw. einstückiges Ausbilden der die Düse begrenzenden Wände auf einem Träger, insbesondere einschließlich des Abschnitts der Düse zwischen der Mündung des Zuführungsröhrchens und der Auslassöffnung der Düse, Anordnen bzw. einstückiges Ausbilden der Blöcke in einem Abstand zueinander auf dem Träger und/oder auf dem Deckel und Anordnen von Träger und Deckel gegeneinander erfolgen. Da sich die Wände und Blöcke jeweils flüssigkeitsdicht zwischen Träger und Deckel erstrecken, bilden Träger, Deckel und die Blöcke das Zuführungsröhrchen zwischen sich. Die Wände bilden mit den ihnen zugewandten Oberflächen der Blöcke den Strömungskanal für die Hüllflüssigkeit. Die Auslassöffnung der Düse wird in einem Abstand von der Mündung des Zuführungsröhrchens von den Wänden, Träger und Deckel gebildet. Die Wandungen der Blöcke und der Abschnitt des an den Blöcken angeordneten Trägers und/oder Deckels, die den Innenquerschnitt des Zuführungsröhrchens aufspannen, können dadurch erzeugt werden, dass von einem auf dem Träger und/oder Deckel angeordneten, insbesondere einstückig mit Träger und/oder Deckel ausgebildeten Großblock das Material entfernt wird, das im Innenquerschnitt des Zuführungsröhrchens angeordnet ist, z.B. durch Fräsen oder Läserschneiden des Großblocks entlang der Längsachse des Zuführungsröhrchens. Alternativ kann eine Düse insbesondere dieser Ausführungsform durch Spritzguß hergestellt werden. Die Einlassöffnung für Hüllstromflüssigkeit kann in einer Wand angeordnet sein, die Träger und Deckel verbindet Vorzugsweise ist zumindest ein generell als Piezokristall bezeichneter Schwingungserzeuger In oder angrenzend an eine Wand bzw. Halter gegenüber der Auslassöffnung der Düse angeordnet.

Bevorzugt bestehen Träger, Blöcke und Deckel, und optional zwischen Träger und Deckel angeordnete Wände, die den Abschnitt der Düse zwischen der Mündung des Zuführungsröhrchens und der Auslassöffnung der Düse bilden, aus Kunststoff.

Das erfindungsgemässe Verfahren ist eines gemäss Anspruch 14 zur Fraktionierung von Partikeln, vorzugsweise zur Herstellung einer Fraktion von Partikeln, insbesondere unter Verwendung der Düse.

Optional enthält das Verfahren den Schritt des Detektierens einer Eigenschaft der Partikel und,
weiter optional, den Schritt des Behandelns (z.B. durch Laserbestrahlung) und/oder des Ablenkens der Partikel enthaltenden Flüssigkeit in getrennte Behälter in Abhängigkeit von einer detektierten Eigenschaft, z.B. mittels eines elektrischen Felds, durch das die Partikel enthaltende Flüssigkeit nach Beaufschlagung mit einer von der detektierten Eigenschaft abhängigen elektrischen Ladung hindurchtritt. Vorzugsweise wird die Düse in Schwingungen versetzt, z.B. mittels Anregung eines Schwingungserzeugers, die zur Bildung eines Stroms partikelhaltiger Tröpfchen führt.

Insbesondere bevorzugt ist das Verfahren zur Herstellung geschlechtschromosomenspezifisch sortierter Spermatozoenfraktionen, z.B. mit dem Schritt des Detektierens des Geschlechtschromosoms, direkt oder indirekt durch Detektion des Gesamt-DNA-Gehalts, der aus der Düse austretenden Säugetlerspermienund Ablenken der Säugetierspermien nach dem detektierten Geschlechtschromosom in getrennte Fraktionen, wie dies generell bekannt ist, z.B. aus US 5,135,759 mit elektrischer Aufladung der aus der Düse austretenden Spermien enthaltenden Flüssigkeitströpfchen und deren Ablenkung zwischen geladenen Platten oder gemäß WO2010/149739 mittels Ablenkung aus der Düse austretender Spermien enthaltender Flüssigkeitströpfchen mittels deren oberflächlicher Verdampfung durch Laserstrahlung.

Das Verfahren kann sich weiter dadurch auszeichnen, dass die Partikel nicht-menschliche Säugetierspermlen sind, die nach Austritt in Flüssigkeitströpfchen mit Kernstromflüssigkeit und Hüllstromflüssigkeit aus der Auslassöffnung der Düse geschlechtschromosomenspezifisch in getrennte Fraktionen abgelenkt werden, in denen die Spermien in Bezug auf das Vorliegen eines Geschlechtschromosoms angereichert sind. Das Verfahren kann sich dadurch auszeichnen, dass Flüssigkeitströpfchen abhängig von einem geschlechtschromosömenspezifischen Detektionssignal mit Laserstrahlung bestrahlt werden, die in einer zweiten Position auf den Fluidstrom gerichtet ist, die In einem größeren Abstand zur Düse als die erste Position des Fluidstroms angeordnet ist, in welcher ein geschlechtschromosomenspezifisches Signal detektiert wird, wobei die Laserstrahlung nur oberflächlich vom Flüssigkeitströpfchen absorbiert wird und das Flüssigkeitströpfchen nur oberflächlich verdampft und in die zur oberflächlichen Verdampfung entgegengesetzte Richtung beschleunigt und abgelenkt wird. Insbesondere kann die Laserstrahlung auf die Oberfläche eines der Flüssigkeitsträpfchen zur Induktion einer oberflächlichen Photodisruption fokussiert werden und/oder die Laserstrahlung kann eine Wellenlänge aufweisen, die vom Flüssigkeitströpfchen absorbiert wird. Alternativ kann das Verfahren die Spermien dadurch In geschlechtschromosomenspezifischen Fraktionen anreichern, dass Flüssigkeitströpfchen abhängig von einem geschlechtschromosomenspezifischen Detektionssignal durch Beaufschlagung der Düse mit elektrischer Ladung elektrisch geladen werden und anschließend mittels Durchtritt durch ein elektrisches Feld in getrennte Fraktionen abgelenkt werden. Das Verfahren ist ein Herstellungsverfahren für ein Präparat von Partikeln, die senkrecht zu ihrer Längsachse einen Querschnitt aufweisen, der in einer ersten Dimension kleiner ist als in einer zweiten Dimension, die insbesondere nicht-menschliche Säugetierspermien sind, wobei die detektierte Eigenschaft das Vorliegen des X-Chromosoms oder des Y-Chromosoms in einem Spermium ist, und das Verfahren das Fraktionieren der Spermien in Abhängigkeit von der detektierten Eigenschaft in zumindest zwei verschiedene Fraktionen umfasst, insbesondere zur Herstellung einer Fraktion, in der die Spermien mit X-Chromosom oder Y-Chromosom angereichert sind, z.B. zu zumindest 90%, bevorzugt zu zumindest 95%, bevorzugter zu zumindest 98% oder zumindest 99%. Das Durchströmen der Düse von Hüllstromflüssigkeit erfolgt vorzugsweise in dem Abschnitt III in einem rotationssymmetrischen innenquerschnitt oder einem Innenquerschnitt aus zwei durch das Zuführungsröhrchen beabstandeten Abschnitten mit jeweils rechteckigem Querschnitt, wobei sich der Innenquerschnitt zwischen Zuführungsröhrchen und Düsenwandung in Richtung auf den zwischen Abschnitt III und der Auslassöffnung angeordneten Abschnitt IV verjüngen kann. Die Abschnitte III und IV können mit jeweils rechteckigem Querschnitt zwischen einem Träger und einem Deckel, die sich beidseitig beabstandet und parallel der Längsachse der Kemströmflüssigkeit erstrecken, gebildet sein und In einem Abstand beidseitig zum Zuführungsröhrchen durch die Wandungen der Düse begrenzt sein. In Abschnitt IV, der sich am zweiten Ende der Düse befindet, verjüngt sich bevorzugt der Querschnitt der Hüllstromflüssigkeit zur am zweiten Ende der Düse angeordneten Auslassöffnung und Ist optional rotationssymmetrisch, bevorzugt kegelförmig bzw. kegelstumpfförmig ausgebildet oder kann einen sich zur Auslassöffnung der Düse verkleinernden rechteckigen Querschnitt aufweisen. Wie mit Bezug auf Abschnitt III beschrieben, kann auch Abschnitt IV Abschnitte mit jeweils rechteckigem Querschnitt aufweisen, die sich beidseitig des Zuführungsröhrchens erstrecken und zwischen dem Träger und dem Deckel gebildet sind und in einem Abstand beidseitig zum Zuführungsröhrchen durch die Wandungen der Düse begrenzt sind. In Abschnitten I, II und III strömt die Kernstromflüssigkeit durch das Zuführungsröhrchen und tritt aus dessen Mündung in den angrenzenden Abschnitt IV ein, in dem die Kernstromflüssigkeit unmittelbar von der Hüllstromflüssigkeit umfasst wird. In Abschnitten I, II und III strömt die Hüllstromflüssigkeit bevorzugt zwischen dem Zuführungsröhrchen und den Wandungen der Düse und in Abschnitt IV zwischen der aus der Mündung des Zuführungsröhrchens austretenden Kernstromflüssigkeit und der Düsenwandung. Das Führen der Kernstromflüssigkeit durch dasZuführungsröhrchen ergibt einen Kernstrom, dessen Querschnitt in einer ersten Dimension kleiner ist als In einer zweiten Dimension, wobei dieser Querschnitt die regelmäßige Ausrichtung von Partikeln mit flachem Querschnitt erzeugt, welcher in einer ersten Dimension kleiner Ist als In einer zweiten Dimension. Bevorzugt weist die von Hüllstromflüssigkeit ummantelte Kernstromflüssigkeit, die durch den Austritt der Kernstromflüssigkeit aus der Mündung des Zuführungsröhrchens gebildet ist, einen Querschnitt auf, welcher In einer ersten Dimension kleiner ist als in einer zweiten Dimension.

Der Abschnitt IV der Düse kann von einem Düseneinsatz gebildet sein. Die Kernstromflüssigkeit tritt in den an die Mündung des Zuführungsröhrchens anschließenden Düsenabschnitt IV bzw. In den Düseneinsatz Im Wesentlichen als ein Strahl mit der Geometrie ein, die dem innenquerschnitt des Zuführungsröhrchens entspricht. Aus der Auslassöffnung des Düseneinsatzes tritt eine von Hüllstromflüssigkeit ummantelte Kernstromflüssigkeit aus. Bei kreisförmiger Auslassöffnung der Düse tritt die Hüllstromflüssigkeit in zylindrischer Geometrie aus der Auslassöffnung aus. Denn auf Grund der Ausrichtung der Partikel in der Kernstromflüssigkeit durch den gestreckten innenquerschnitt des Zuführungsröhrchens kann die Düse einen einfachen, rotationssymmetrischen, kegelstumpfförmigen Abschnitt IV aufweisen. In der Hüllstromflüssigkeit ist die Kernstromflüssigkeit mit gestrecktem Querschnitt angeordnet, In welcher die Partikel Im Wesentlichen gleichförmig ausgerichtet sind, z.B. mit den Längsseiten ihres Querschnitts parallel zu den Längsseiten des Querschnitts der Kernstromflüssigkeit und mit den Schmalseiten ihres Querschnitts parallel zu den Schmalseiten des Querschnitts der Kernstromflüssigkeit, wobei bevorzugt die Längsachse der Partikel, z.B. von Spermien, parallel zur Längsachse der Kernstromflüssigkeit im Zuführungsröhrchen angeordnet ist.

In einer bevorzugten Ausführungsform läuft der Düsenabschnitt IV in dem dem zweiten Ende der Düse zugewandten Ende kegelstumpfförmig auf die Auslassöffnung zu, während der Querschnitt des Zuführungsröhrchens über seine gesamte Länge, zumindest in seinem Abschnitt III konstant bleibt. Insbesondere bei einer Strömungsgeschwindigkeit der Hüllflüssigkeit in dem Querschnitt auf Höhe der Mündung des Zuführungsröhrchens gleich oder größer als die Strömungsgeschwindigkeit der Kernflüsigkeit resultiert aus dem sich verringernden Innenquerschnitt der Düse Im Abschnitt IV eine Beschleunigung der Hüllstromflüssigkeit gegenüber der Kernstromflüssigkeit, wodurch eine Vereinzelung von Partikeln der Kernstromftüssigkeit entlang der Längsachse des Düseneinsatzes erfolgt.

In einer Ausführungsform wird die Kernstromflüssigkeit etwa mit dem gleichen Volumenstrom in die erste Einlassöffnung gepumpt wie die Hüllstromflüssigkeit in die zweite Einlassöffnung gepumpt wird. Alternativ kann die Kernstromflüssigkeit auch mit einem Volumenstrom in die erste Einlassöffnung gepumpt werden, der größer oder kleiner ist als der Volumenstrom, mit dem die Hüllstromflüssigkeit in die zweite Einlassöffnung gepumpt wird.

Bevorzugt weist die Düse einen Schwingungserzeuger, z. B. ein Piezoelement auf, der eingerichtet ist, senkrecht zur Längsachse der Düse verlaufende Druckwellen zu erzeugen, um im Flüssigkeitsstrom nach der Auslassöffnung einen Tropfenstrom zu erzeugen. Der Schwingungserzeuger kann z.B. in einer Wand am ersten Ende der Düse angebracht sein, insbesondere eingegossen sein, z.B. in einen Halter.

Die Düse kann an einem Durchflusszytometer angeordnet sein, welches zumindest eine erste Strahlungsquelle aufweist, die auf einen ersten Abschnitt des aus der Auslassöffnung austretenden Flüssigkeitsstroms gerichtet ist, z.B. einen Laser, und einen gegenüber der Strahlungsquelle auf den ersten Abschnitt des Flüssigkeitsstroms gerichteten ersten Detektor, wobei optional der Detektor ein Signal erzeugt, das eine Auslenkungseinrichtung ansteuert, um Abschnitte des Flüssigkeitsstroms in Abhängigkeit von der Detektion mittels des Signals abzulenken, z.B. zu fraktionieren. Optional ist eine zweite Strahlungsquelle z.B. auf einen zweiten Abschnitt des Flüssigkeitsstroms zwischen der ersten Strahlungsquelle und dem Düseneinsatz gerichtet, während ein zweiter Detektor auf diesen zweiten Abschnitt gerichtet ist. Bevorzugt erzeugt der zweite Detektor ein zweites Signal, das die Auslenkungseinrichtung steuert, so dass Abschnitte des Flüssigkeitsstroms zusätzlich in Abhängigkeit von dem zweiten Signal ausgelenkt werden.

Die Düse kann eine Metall- und insbesondere eine Edelstahlverbindung zur Ladungsübertragung aufweisen, welche bevorzugt in Abschnitt I der Düse am Träger beidseitig der Längsachse angeordnet ist. Die Edelstahlverbindung weist einen elektrischen Kontakt auf, der die Zuleitung für Kernstromflüssigkeit und/oder die Zuleitung für Hüllstromflüssigkeit mit elektrischer Ladung beaufschlagen kann. Vorzugsweise wird der elektrische Kontakt in Abhängigkeit von einem ersten und/oder zweiten Signal gesteuert, welches durch Messung der vereinzelten Tröpfchen unterhalb der Auslassöffnung erhalten wird. In Abhängigkeit vom ersten und/oder zweiten Signal erfolgt eine positive oder negative Aufladung der Tröpfchen. Entsprechend ihrer Ladung können die Tropfen in unterschiedliche Richtungen abgelenkt werden, sodass mindestens zwei Fraktionen erhalten werden.

### Genaue Beschreibung der Erfindung

Die Erfindung wird nun genauer mit Bezug auf die Figuren und anhand von Beispielen beschrieben, die schematisch
- in Figur 1 eine erfindungsgemäße Düse im Querschnitt,
- in den Figuren 2 bis 5 Schnitte entlang der Linie A-A in Figur 1,
- in Figur 6 eine Aufsicht auf eine bevorzugte Ausführungsform der Düse ohne Deckel,
- in Figur 7 einen Deckel für die Düse nach Figur 6,
- in Figur 8 eine Aufsicht auf eine weitere bevorzugte Ausführungsform der Düse ohne Deckel,
- in Figur 9 einen Deckel für die Düse nach Figur 8 und
- in Figur 10 eine weitere Ausführungsform der Düse zeigen.

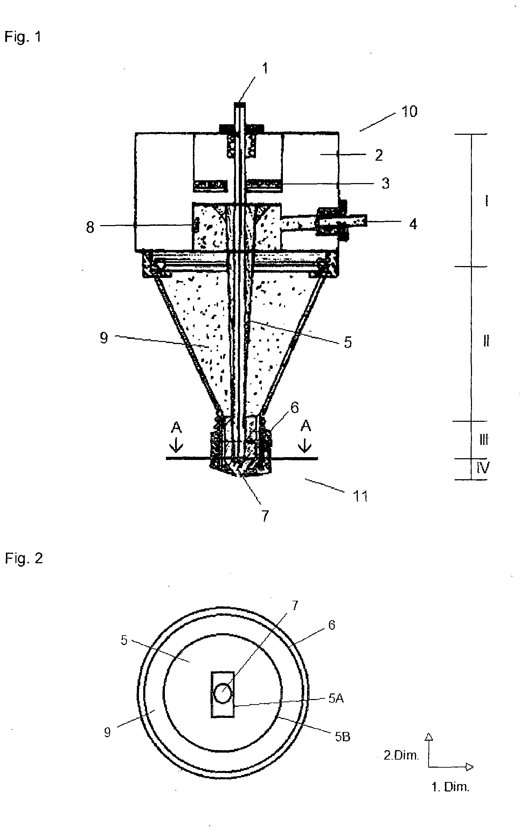

In den Figuren bezeichnen gleiche Bezugsziffern funktionsgleiche Elemente. Die Figur 1 zeigt eine erfindungsgemäße Düse zur Partikelausrichtung für ein Durchflusszytometer mit einem ersten Ende 10 und einem zweiten Ende 11. Am ersten Ende 10 der Düse befindet sich Abschnitt I, welcher vorliegend zylindrisch ausgebildet ist und im Wesentlichen von einem Halter 2 gebildet wird, entlang dessen Längsachse das Zuführungsröhrchen 5 für Kernstromflüssigkeit verläuft und in dem eine zweite Einlassöffnung 4 für Hüllstromflüssigkeit eingelassen ist. Der angrenzende Abschnitt II ist koaxial zu Abschnitt I angeordnet und verjüngt sich zum zweiten Ende 11 der Düse hin konisch. Der angrenzende zylinderförmige Abschnitt III wird durch einen Düseneinsatz 6 gebildet, welcher am zweiten Ende 11 der Düse angeordnet ist. Koaxial zu dem durch den Düseneinsatz 6 gebildeten Abschnitt III verläuft in dessen Innenvolumen das Zuführungsröhrchen 5 für Kernstromflüssigkeit. Die Mündung 12 des Zuführungsröhrchens 5 liegt z.B. zwischen Abschnitt III und Abschnitt IV. Abschnitt IV wird auch von dem Düseneinsatz 6 gebildet, ist kegelstumpfförmig und verjüngt sich in Richtung des zweiten Endes 11 der Düse bis zu einer Auslassöffnung 7 mit rundem Querschnitt.

Eine erste Einlassöffnung 1 für Kernstromflüssigkeit befindet sich am ersten Ende 10 der Düse und ist in den Halter 2 eingelassen. In der gezeigten Ausführungsform ist die erste Einlassöffnung 1 für Kernstromflüssigkeit koaxial im zylinderförmigen Abschnitt der Düse eingelassen und wird von dem Zuführungsröhrchen 5 gebildet. Dieses Zuführungsröhrchen 5 verläuft vom ersten Ende 10 der Düse in Richtung des zweiten Endes 11 der Düse, wo es in den Düseneinsatz 6 führt und eine Mündung 12 in einem Abstand zur Auslassöffnung 7 aufweist. Das Zuführungsröhrchen 5 für Kernstromflüssigkeit und der Träger 2 sind koaxial angeordnet.

Am Halter 2 ist beabstandet zu der ersten Einlassöffnung 1 für Kernstromflüssigkeit eine zweite Einlassöffnung 4 für Hüllstromflüssigkeit angeordnet. Diese zweite Einlassöffnung 4 befindet sich in der gezeigten Ausführungsform in der Wand des Halters 2. An die zweite Einlassöffnung 4 ist die Hüllflüssigkeitszufuhr 9 angeschlossen, welche den Halter 2 mit dem am zweiten Ende 11 der Düse angeordneten Düseneinsatz 6 verbindet. Die Hüllflüssigkeitszufuhr 9 ist in dem Bereich zwischen Halter 2 und Düseneinsatz 6 kegelstumpfförmig ausgebildet. Das Zuführungsröhrchen 5 verläuft koaxial zu der Hüllflüssigkeitszufuhr 9 in deren Innenvolumen.

Sowohl die Hüllflüssigkeitszufuhr 9 als auch das Zuführungsröhrchen 5 für Kernstromflüssigkeit münden in einen Düseneinsatz 6, welcher am zweiten Ende 11 der Düse angeordnet ist. Der Düseneinsatz 6 weist angrenzend an seine Auslassöffnung 7, welche kreisförmig gestaltet ist, einen sich verjüngenden Abschnitt auf. Das Zuführungsröhrchen 5 für Kernstromflüssigkeit ist koaxial zum Düseneinsatz 6 angeordnet und endet in einem Abstand zur Auslassöffnung 7. Der Zwischenraum zwischen Zuführungsröhrchen 5 und Wandung des Düseneinsatzes 6 wird mit Hüllstromflüssigkeit ausgefüllt.

An der Düse sind außerdem ein Piezokristall 3 zur Erzeugung eines Tröpfchenstroms sowie eine Metall- bzw. Edelstahlverbindung 8 zur Ladungsübertragung auf die Hüllstromflüssigkeit angeordnet.

Die Figur 2 zeigt einen Schnitt entlang der in Figur 1 gezeigten Linie A-A und die in einem Abstand von der Mündung 12 angeordnete Auslassöffnung 7 der Düse. Der Innenquerschnitt 5A des Zuführungsröhrchens 5 für Kernstromflüssigkeit wird in einer ersten Dimension und einer zweiten Dimension aufgespannt, welche sich betragsmäßig deutlich voneinander unterscheiden, vorliegend etwa um den Faktor 0,5. Gemäß dieser Ausführungsform weist der Innenquerschnitt 5A die Form eines Rechtecks auf. Die anderen Querschnitte sind im Wesentlichen kreisförmig.

Die Hütistromftüssigkeitszufuhr 9 füllt den Raum zwischen der Innenwandung des Düseneinsatzes 6 und der Außenwandung des Zuführungsröhrchens 5 aus und kontaktiert die Kernstromflüssigkeit in dem Abschnitt zwischen der Mündung 12 und der beabstandeten Auslassöffnung 7.

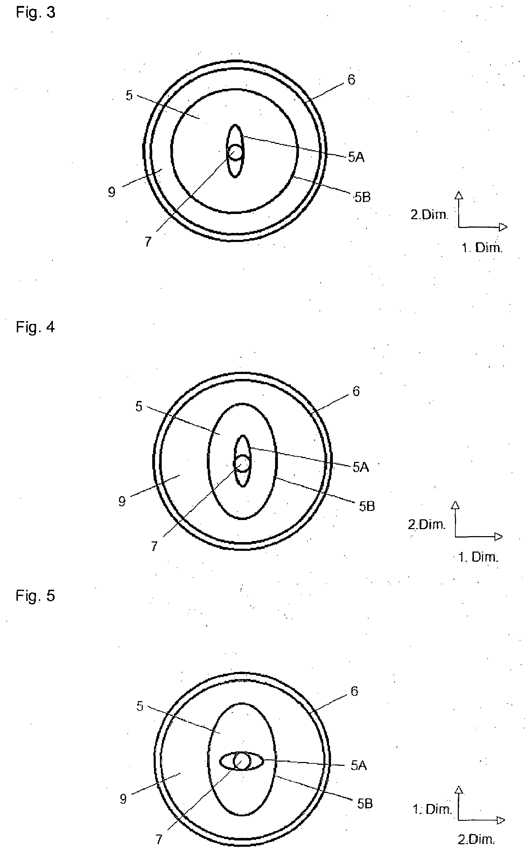

Die Figur 3 zeigt einen Schnitt entlang der Linie A-A von Figur 1. Der innenquerschnitt 5A des Zuführungsröhrchens 5 wird in einer ersten Dimension und einer zweiten Dimension aufgespannt, welche sich betragsmäßig deutlich unterscheiden, vorliegend etwa um den Faktor 0,3. Gemäß dieser Ausführungsform weist der innenquerschnitt 5A die Form einer Ellipse auf. Der Außenquerschnitt 5B des Zuführungsröhrchens 5, der Düseneinsatz 6, die Hüllflüssigkeitszufuhr 9 und die in einem Abstand von der Mündung 12 angeordnete Auslassöffnung 7 weisen einen kreisförmigen Querschnitt auf.

Die Figur 4 zeigt einen Schnitt A-A von Figur 1 und die in einem Abstand von der Mündung 12 angeordnete Auslassöffnung 7 der Düse. Der Innenquerschnitt 5A des Zuführungsröhrchens 5 ist in einer ersten Dimension etwa um den Faktor 0,3 kleiner als in einer zweiten Dimension und weist die Form einer Ellipse auf. Auch der Außenquerschnitt 5B des Zuführungsröhrchens 5 ist in einer ersten Dimension kleiner als in einer zweiten Dimension und weist die Form einer Ellipse auf. Die Längsachse des elliptischen Innenquerschnitts 5A liegt parallel zur Längsachse des elliptischen Außenquerschnitts 5B.

Die Figur 5 zeigt einen Schnitt A-A von Figur 1 und die in einem Abstand von der Mündung 12 angeordnete Auslassöffnung 7 der Düse. Der Innenquerschnitt 5A des Zuführungsröhrchens 5 weist die Form einer Ellipse auf und wird in einer ersten Dimension und einer zweiten Dimension aufgespannt, welche sich vorliegend etwa um den Faktor 0,3 unterscheiden. Der Außenquerschnitt 5B weist ebenfalls die Form einer Ellipse auf und wird in einer ersten und einer zweiten Dimension aufgespannt. Beide Ellipsen sind so angeordnet, dass ihre Längsachsen rechtwinklig zueinander sind.

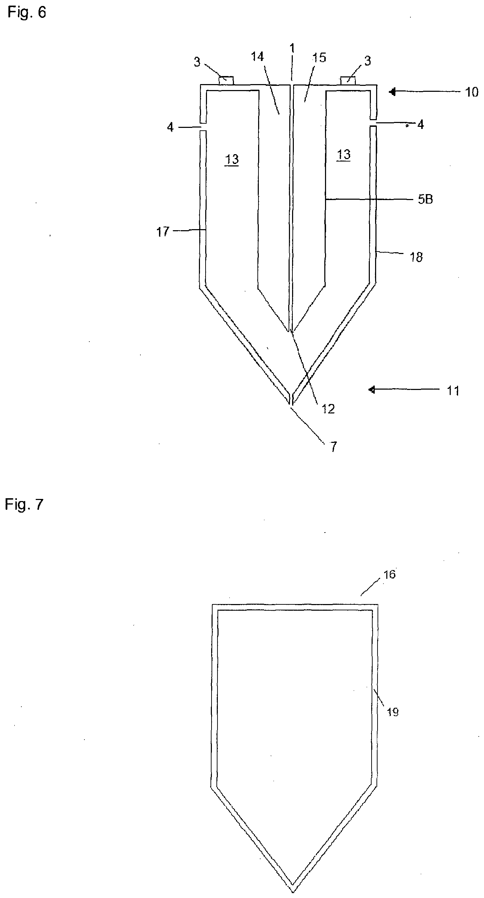

In Figur 6 ist eine bevorzugte Ausführungsform gezeigt, bei der die Düse einen Träger 13 und zwei darauf angeordnete Blöcke 14, 15 aufweist, die in einem Abstand zueinander angeordnet sind. Die Blöcke 14, 15 begrenzen mit dem Träger 13 und einem gegenüber des Trägers 13 angeordneten Deckel 16, der in Figur 7 gezeigt ist, das Zuführungsröhrchen 5. Die einander zugewandten Oberflächen des Trägers 13 und des Deckels 16 sind vorzugsweise parallel und plan. Der Träger 13 und der Deckel 16 können jeweils plattenförmig sein.

Die dem Zuführungsröhrchen 5 gegenüberliegenden Oberflächen der Blöcke 14, 15 bilden den Teil des Außenquerschnitts 5B des Zuführungsröhrchens 5, der mit den Wänden 17, 18 den Strömungsweg für die Hüllstromflüssigkeit begrenzt. Die Wände 17, 18 können generell zueinander parallel sein und die Oberflächen der Blöcke 14, 15, die den Wänden 17, 18 zugewandt sind, können parallel zu den Wänden verlaufen. Der Abschnitt der Düse zwischen der Mündung 12 und der Auslassöffnung 7 kann von einem Düseneinsatz 6 gebildet sein, der flüssigkeitsdicht an die Wände 17, 18 anschließt. Der Düseneinsatz 6 kann entsprechend Figur 6 abschnittsweise innerhalb der Wände 17, 18 angeordnet sein oder außerhalb, wobei vorzugsweise jeweils ein Vorsprung 20 auf der Innenoberfläche der Wände 17, 18 bzw. auf deren Außenoberfläche angebracht ist, wobei sich der Vorsprung 20 vorzugsweise über den Träger 13 und den Deckel 16 erstreckt. Die Wände 17, 18 sind auf dem Träger 13 angeordnet.

Der Deckel 16 erstreckt sich bis an den flüssigkeitsdicht angeordneten Düseneinsatz 6.

Optional kann zwischen Wänden 17, 18 und Träger 13 bzw. Deckel 16 eine Dichtung 19 angeordnet sein. Weiter kann zwischen den Blöcken 14, 15 und dem Deckel 16 eine Dichtung 19 angeordnet sein, vorzugsweise ist der Deckel 16 plan und weist eine elastische Oberfläche als Dichtung 19 auf, die sich über den Teil seiner Oberfläche erstreckt, der gegen die dem Deckel 16 zugewandten Stirnseiten der Wände 17, 18 angeordnet ist und den Teil seiner Oberfläche, die gegen die Blöcke 14, 15 angeordnet ist, insbesondere über die vollständige Oberfläche des Deckels 16, der sich über ihm zugewandten Stirnseiten der Wände 17, 18 erstreckt.

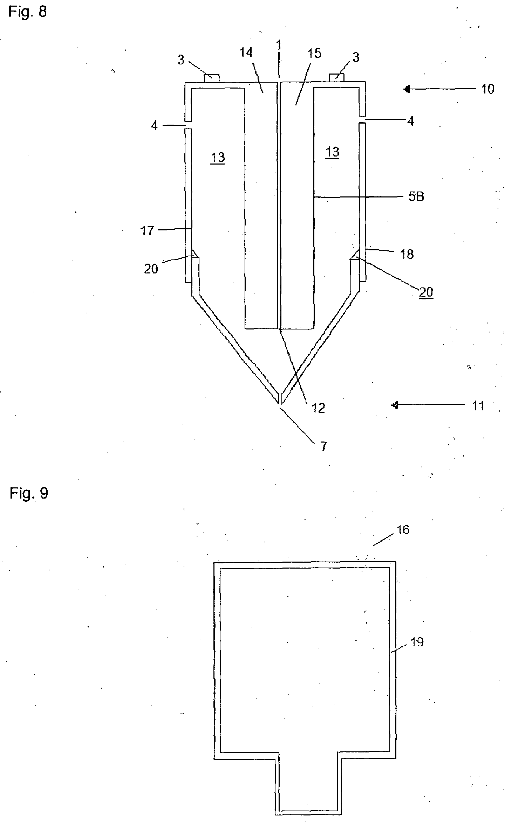

Die Figur 8 zeigt eine generell bevorzugte Ausführungsform der Düse, bei der das Zuführungsröhrchen 5 durch zwei beabstandete parallele Blöcke 14, 15 zwischen der planen Oberfläche des Trägers 13 und der parallelen und planen beabstandeten Oberfläche des Deckels 16 gebildet ist, bei der sich zwischen Träger 13 und Deckel 16 zu den Blöcken 14, 15 beabstandete Wände 17, 18 erstrecken, die sich zu einer von der Mündung 12 des Zuführungsröhrchens 5 beabstandeten Auslassöffnung 7 verjüngen. Wie in Figur 8 gezeigt, kann der Abschnitt der Düse, der sich von der Mündung 12 des Zuführungsröhrchens 5 zur Auslassöffnung 7 verjüngt, von den Wänden 17, 18 gebildet werden, die sich flüssigkeitsdicht zwischen Träger 13 und Deckel 6 erstrecken. Die zweite Einlassöffnung 4 für Hüllstromflüssigkeit ist in einer der Wände 17, 18 angeordnet, die erste Einlassöffnung 1 für Kernstromflüssigkeit am ersten Ende 10 der Düse. Der in Figur 9 gezeigte Deckel 16 kann durch Anordnung gegen die Stirnflächen der Blöcke 14, 15 und der Wände 17, 18, insbesondere mit einer zwischenliegenden Dichtung 19, den Querschnitt zwischen Deckel 16 und Träger 13 sowie zwischen Wänden 17, 18 und Blöcken 14, 15 zu zwei rechteckigen Innenquerschnitten für die Hüllflüssigkeit begrenzen, z.B. bis angrenzend an einen im Abschnitt IV angeordneten Düseneinsatz.

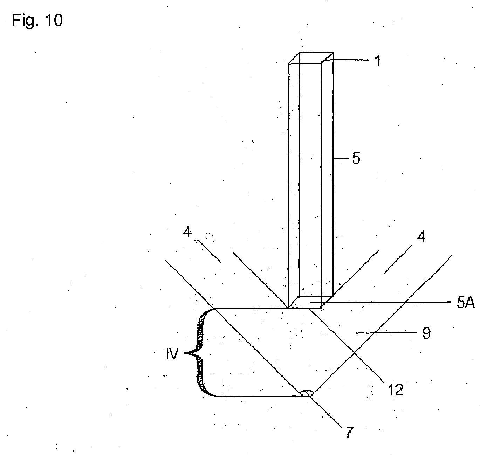

Figur 10 zeigt eine Ausführungsform, bei der die Mündung 12 des Zuführungsröhrchens 5 in einer Düse angeordnet ist, die in der Längsachse des Zuführungsröhrchens 5 auf den Abschnitt IV zwischen der Mündung 12 des Zuführungsröhrchens 5 und ihrer Auslassöffnung 7 begrenzt ist. Der Abschnitt IV verjüngt sich von der Mündung 12 zur Auslassöffnung 7. Zweite Einlassöffnungen 4 für Hüllstromflüssigkeit sind in der Ebene der Mündung 12 des Zuführungsröhrchens 5 angeordnet. Der Innenquerschnitt 5A des Zuführungsröhrchens 5 endet in der Mündung 12.

### Beispiel 1: Detektion Y-Chromosomen-haltiger Spermien in Frischsamen und geschlechtsspezifische Sortierung

Frisch gewonnener Bullensamen wurde in üblicher Weise in Verdünner verdünnt und mit DNA-spezifischem Farbstoff, z.B. Bisbenzimid H 33342 (Hoechst), für 30 bis 60 min bei einer Temperatur von 20 °C bis 40 °C inkubiert und anschließend in einem Durchflusszytometer gemäß US 5125759 oder der DE 10 2005 044 530 mit Licht der Anregungswellenlänge für den Farbstoff bestrahlt. Die jeweilige Emission wurde gemessen.

Die Ausrichtung der Spermien wurde mit einem Detektor bestimmt, der unmittelbar stromabwärts der Auslassöffnung auf den austretenden Flüssigkeitsstrom aus vereinzelten Tropfen gerichtet war. Der Gesamt-DNA-Gehalt wurde mit einem weiteren Detektor bestimmt, der weiter stromabwärts auf den Flüssigkeitsstrom gerichtet war. Die Ablenkungseinrichtung wies zwei entgegengesetzt geladene Platten beidseitig des Flüssigkeitsstroms auf und einen Kontakt zur elektrischen Aufladung der Flüssigkeit in der Düse. Diese Aufladung wurde wie bekannt in Abhängigkeit von dem Signal des Detektors aufgegeben, der die Ausrichtung der Spermien bestimmt, und die Polarität der Aufladung in Abhängigkeit von dem Signal des Detektors zur Bestimmung des Gesamt-DNA-Gehalts. Auf diese Weise wurden die Spermatozoen abhängig von dem detektierten Signal durch ein elektrisches Feld in geschlechtschromosomspezifische Fraktionen abgelenkt.

Optional wurde ein Fluorid zur Immobilisierung der Spermien zugesetzt, z.B. in die während des Sortierverfahrens verwendete Hüll- oder Transportflüssigkeit, und/oder vor oder während des Zusatzes des Farbstoffs, um die Penetration des Farbstoffs in die Spermatozoen zu erhöhen. Fluoridionen wurden im Bereich von 0,1 bis 100 mM, vorzugsweise von 10 nM bis 10 mM zugesetzt. Es wurde gefunden, dass die optimale Konzentration des Fluorids, z.B. NaF oder KF, zwischen verschiedenen Spezies und für Individuen abwich. Die optimale Konzentrationen für die Spezies ist spezifisch und konnte allgemein als die Konzentration bestimmt werden, die bei mikroskopischer Betrachtung eine Immobilisierung von wenigstens 90% der Spermatozoen ergab, vorzugsweise von im wesentlichen allen Spermatozoen. Entsprechend bezieht sich die vorliegende Erfindung auch auf Zusammensetzungen der mit dem erfindungsgemäßen Verfahren hergestellten Spermafraktionen, und auf Verfahren zur Herstellung von geschlechtsspezifischen Spermafraktionen und anschließenden Konservierung der Spermafraktionen von nicht-menschlichen Säugetieren, jeweils vorzugsweise in Anwesenheit von Fluorid und/oder Antioxidationsmitteln.

**Bezugszeichenliste**

| | | | |
|---|---|---|---|
| | | 8 | Edelstahlverbindung zur Ladungsübertragung |
| 1 | Erste Einlassöffnung | 9 | Hüllflüssigkeitszufuhr |
| 2 | Halter | 10 | Erstes Ende der Düse |
| 3 | Piezokristall | 11 | Zweites Ende der Düse |
| 4 | Zweite Einlassöffnung | 12 | Mündung |
| 5 | Zuführungsröhrchen | 13 | Träger |
| 5A | Innenquerschnitt des Zuführungsröhrchens | 14 | Block |
| | | 15 | Block |
| 5B | Außenquerschnitt des Zuführungsröhrchens | 16 | Deckel |
| | | 17 | Wand |
| 6 | Düseneinsatz | 18 | Wand |
| 7 | Auslassöffnung | 19 | Dichtung |
| | | 20 | Vorsprung |

## Patentansprüche

1. Düse zur Partikelausrichtung für ein Durchflusszytometer mit einem ersten Ende (10) und einem gegenüberliegenden zweiten Ende (11) und einem mit seiner Mündung (12) im Innenvolumen der Düse angeordneten Zuführungsröhrchen (5) für Kernstromflüssigkeit, das in einem an seine Mündung (12) angrenzenden Abschnitt auf einer gemeinsamen Achse mit der Düse angeordnet ist, wobei das Zuführungsröhrchen (5) eine erste Einlassöffnung (1) für Kernstromflüssigkeit aufweist und seine Mündung (12) zu einer am zweiten Ende (11) der Düse angeordneten Auslassöffnung (7) beabstandet ist und die Düse eine zweite Einlassöffnung (4) für Hüllstromflüssigkeit aufweist, wobei sich der an die Mündung des Zuführungsröhrchens (5) angrenzende Abschnitt über die vollständige Länge des Zuführungsröhrchens (5) erstreckt, die innerhalb der Düse angeordnet ist und der Innenquerschnitt (5A) des Zuführungsröhrchens (5) für Kernstromflüssigkeit senkrecht zur Längsachse des Zuführungsröhrchens (5) ist, **dadurch gekennzeichnet, dass** der Innenquerschnitt (5A) des Zuführungsröhrchens (5) zumindest in dem an seine Mündung (12) angrenzenden Abschnitt in einer ersten Dimension um zumindest den Faktor 0,3 kleiner ist als in einer zweiten Dimension und dass der Innenquerschnitt des Zuführungsröhrchens (5) rechteckig oder elliptisch ist und in der ersten Dimension 50µm bis 200µm und in der zweiten Dimension 75µm bis 300µm beträgt und wobei der Innenquerschnitt (5A) des Zuführungsröhrchens (5) vom ersten Ende (10) bis zum zweiten Ende (11) der Düse konstant bleibt.

2. Düse nach Anspruch 1, **dadurch gekennzeichnet, dass** das Zuführungsröhrchen (5) einen rechteckigen Innenquerschnitt (5A) aufweist, dessen Längserstreckung in der zweiten Dimension liegt.

3. Düse nach Anspruch 1, **dadurch gekennzeichnet, dass** das Zuführungsröhrchen (5) einen ovalen Innenquerschnitt (5A) aufweist, dessen Längserstreckung in der zweiten Dimension liegt.

4. Düse nach einem der voranstehenden Ansprüche, **dadurch gekennzeichnet, dass** das Innenvolumen der Düse entlang ihrer Längsachse auf den Abschnitt zwischen der Auslassöffnung (7) der Düse und der Mündung (12) des Zuführungsröhrchens (5) begrenzt ist.

5. Düse nach einem der voranstehenden Ansprüche, **dadurch gekennzeichnet, dass** ein Piezokristall (3) in einem Abstand zum ersten Ende (10) der Düse an einer Wand angeordnet ist, die den Querschnitt der Düse bis auf das Zuführungsröhrchen (5) verschließt.

6. Düse nach einem der voranstehenden Ansprüche, **dadurch gekennzeichnet, dass** die Düse einen elektrischen Anschluss aufweist, der das Innenvolumen der Düse kontaktiert.

7. Düse nach einem der voranstehenden Ansprüche, **dadurch gekennzeichnet, dass** das Zuführungsröhrchen (5) innerhalb der Düse eine konstante Wandstärke aufweist.

8. Düse nach einem der voranstehenden Ansprüche, **dadurch gekennzeichnet, dass** das Zuführungsröhrchen (5) durch einen Träger (13), einen vom Träger (13) beabstandeten Deckel (16) und zwei zwischen Träger (13) und Deckel (16) angeordneten voneinander beabstandeten Blöcken (14, 15) gebildet wird.

9. Düse nach einem der voranstehenden Ansprüche, **dadurch gekennzeichnet, dass** das Innenvolumen der Düse von einem Träger (13) und einem von diesem beabstandeten Deckel (16) und Wänden (17, 18) begrenzt wird, die sich beabstandet vom Zuführungsröhrchen zwischen Träger (13) und Deckel (16) erstrecken.

10. Düse nach Anspruch 9, **dadurch gekennzeichnet, dass** die Wände (17, 18) entlang der Längsachse des Zuführungsröhrchens (5) parallel zu den Oberflächen der Blöcke (14, 15) angeordnet sind, die den Wänden (17, 18) zugewandt sind.

11. Düse nach einem der Ansprüche 8 bis 10 **dadurch gekennzeichnet, dass** die einander zugewandten Oberflächen von Träger (13) und Deckel (16) parallel und plan sind.

12. Düse nach einem der Ansprüche 8 bis 11, **dadurch gekennzeichnet, dass** die Wände (17, 18) in einem Abschnitt der Düse, der sich zwischen der Mündung (12) des Zuführungsröhrchens (5) und der Auslassöffnung (7) erstreckt, aufeinander zulaufen und zwischen sich die Auslassöffnung (7) bilden.

13. Düse nach einem der Ansprüche 1 bis 11, **dadurch gekennzeichnet, dass** sich die Düse in einem Abschnitt (III) zu der am zweiten Ende (11) der Düse angeordneten Auslassöffnung (7) verjüngt und von einem flüssigkeitsdicht angeordneten Düseneinsatz (6) gebildet ist.

14. Verfahren zur Ausrichtung von Partikeln, die senkrecht zu ihrer Längsachse einen Querschnitt aufweisen, der in einer ersten Dimension kleiner ist als in einer zweiten Dimension, in einer von Hüllstromflüssigkeit ummantelten partikelhaltigen Kernstromflüssigkeit mit den Schritten: Bereitstellen einer Kernstromflüssigkeit und einer Hüllstromflüssigkeit, Pumpen der Kernstromflüssigkeit durch eine erste Einlassöffnung (1) in ein Zuführungsröhrchen (5) und Pumpen der Hüllflüssigkeit durch eine zweite Einlassöffnung (4) und Austretenlassen einer von Hüllstromflüssigkeit ummantelten Kernstromflüssigkeit an dem dem ersten Ende (10) der Düse gegenüberliegenden zweiten Ende (11) einer Düse durch eine Auslassöffnung (7), wobei die Kernstromflüssigkeit in dem Zuführungsröhrchen (5) geführt wird, das in einem an seine Mündung (12) angrenzenden Abschnitt auf einer gemeinsamen Achse mit der Düse angeordnet ist und der Innenquerschnitt (5A) des Zuführungsröhrchens (5) senkrecht zur Längsachse des Zufuhrungsröhrchens (5) ist, wobei sich der an die Mündung des Zuführungsröhrchens (5) angrenzende Abschnitt über die vollständige Länge des Zuführungsröhrchens (5) erstreckt, die innerhalb der Düse angeordnet ist, **dadurch gekennzeichnet, dass** der Innenquerschnitt (5A) des Zuführungsröhrchens (5) zumindest in dem an seine Mündung (12) angrenzenden Abschnitt in einer ersten Dimension um zumindest den Faktor 0,3 kleiner ist als in einer zweiten Dimension und dass der Innenquerschnitt des Zuführungsröhrchens (5) rechteckig oder elliptisch ist und in der ersten Dimension 50µm bis 200µm und in der zweiten Dimension 75µm bis 300µm beträgt und wobei der Innenquerschnitt (5A) des Zuführungsröhrchens (5) vom ersten Ende (10) bis zum zweiten Ende (11) der Düse konstant bleibt.

15. Verfahren nach Anspruch 14 zur Herstellung eines Präparats von Partikeln, die senkrecht zu ihrer Längsachse einen Querschnitt aufweisen, der in einer ersten Dimension kleiner ist als in einer zweiten Dimension, **dadurch gekennzeichnet, dass** die Partikel nicht-menschliche Säugetierspermien sind, mit dem Schritt des Detektierens einer Eigenschaft der Partikel und dem Schritt des Behandelns der Partikel in Abhängigkeit von der detektierten Eigenschaft und/oder dem Schritt des Ablenkens der Partikel in Abhängigkeit von der detektierten Eigenschaft in getrennte Behälter zur Erzeugung zumindest zweier verschiedener Fraktionen.

## Claims

1. Nozzle for the orientation of particles for a flow cytometer having a first end (10) and an opposite second end (11) and an introduction tube (5) for core stream fluid having its orifice (12) arranged in the inner volume of the nozzle, the introduction tube (5) being arranged in a section adjacent its orifice (12) on a common axis with the nozzle, wherein the introduction tube (5) has a first inlet opening (1) for core stream fluid and its orifice (12) is spaced to an outlet opening (7) arranged at a second end (12) of the nozzle and the nozzle having a second introduction opening (4) for sheath stream fluid, wherein the section adjacent the orifice of the introduction tube (5) extends over the entire length of the introduction tube (5) arranged within the nozzle and the inner cross-section (5A) of the introduction tube (5) for core stream fluid is perpendicular to the longitudinal axis of the introduction tube (5), **characterized in that** the inner cross-section (5A) of the introduction tube (5) at least in the section adjacent its orifice (12) in a first dimension is smaller by at least a factor of 0.3 than in a second dimension and that the inner cross-section of the introduction tube (5) is rectangular, or elliptic and in the first dimension is 50 µm to 200 µm and in the second dimension 75 µm to 300 µm and wherein the inner cross-section (5A) of the introduction tube (5) remains constant from the first end (10) to the second end (11).

2. Nozzle according to claim 1, **characterized in that** the introduction tube (5) has a rectangular inner cross-section (5A), the longitudinal extension of which lies in the second dimension.

3. Nozzle according to claim 1, **characterized in that** the introduction tube (5) has an oval inner cross-section (5A), the longitudinal extension of which lies in the second dimension.

4. Nozzle according to one of the preceding claims, **characterized in that** the inner volume of the nozzle along its longitudinal axis is limited to the section between the outlet opening (7) of the nozzle and the orifice (12) of the introduction tube (5).

5. Nozzle according to one of the preceding claims, **characterized in that** a piezo crystal (3) is arranged on a wall with a spacing from the first end (10) of the nozzle, the wall closing the cross-section of the nozzle except for the introduction tube (5).

6. Nozzle according to one of the preceding claims, **characterized in that** the nozzle has an electrical contact that contacts the inner volume of the nozzle.

7. Nozzle according to one of the preceding claims, **characterized in that** the introduction tube (5) within the nozzle has a constant wall thickness.

8. Nozzle according to one of the preceding claims, **characterized in that** the introduction tube (5) is formed by a carrier (13), a lid (16) spaced apart from the carrier (13) and two blocks (14, 15) arranged between the carrier (13) and the lid (16), and spaced apart from one another.

9. Nozzle according to one of the preceding claims, **characterized in that** the inner volume of the nozzle is limited by a carrier (13) and a lid (16) spaced therefrom and walls (17, 18), which with a spacing extend from the introduction tube between carrier (13) and lid (16).

10. Nozzle according to claim 9, **characterized in that** the walls (17, 18) are arranged along the longitudinal axis of the introduction tube (5) in parallel to the surfaces of the blocks (14, 15) facing the walls (17, 18).

11. Nozzle according to one of claims 8 to 10, **characterized in that** the surfaces of the carrier (13) and the lid (16) facing one another are parallel and plane.

12. Nozzle according to one of claims 8 to 11, **characterized in that** the walls (17, 18), run towards one another in a section of the nozzle that extends between the orifice (12) of the introduction tube (5) and the outlet opening (7), and between them form the outlet opening (7).

13. Nozzle according to one of claims 1 to 11, **characterized in that** in a section (III) the nozzle tapers towards the outlet opening (7) arranged at the second end (11) of the nozzle and is formed by a fluid-tight arranged nozzle inset (6).

14. Process for orienting particles which in perpendicular to their longitudinal axis have a cross-section which in a first dimension is smaller than in a second dimension in a particle containing core stream fluid enveloped by a sheath stream fluid by the steps of: Providing a core stream fluid and a sheath stream fluid, pumping of the core stream fluid through a first introduction opening (1) into an introduction tube (5) and pumping the sheath stream fluid through a second introduction opening (4) and exiting of the core stream fluid enveloped by sheath stream fluid at a second end (11) of a nozzle opposite the first end (10) of the nozzle through an outlet opening (7), wherein the core stream fluid is guided in the introduction tube (5) which in a section adjacent its orifice (12) is arranged on a common axis with the nozzle and the inner cross-section (5A) of the introduction tube (5) is perpendicular to the longitudinal axis of the introduction tube (5), wherein the section adjacent the orifice of the introduction tube (5) extends over the entire length of the introduction tube (5) arranged within the nozzle, **characterized in that** the inner cross-section (5A) of the introduction tube (5) at least in the section adjacent its orifice (12) in a first dimension is smaller by at least a factor of 0.3 than in its second dimension and that the inner cross-section of the introduction tube (5) is rectangular or elliptical and in the first dimension is 50 µm to 200 µm and in the second dimension is 75 µm to 300 µm, and wherein the inner cross-section (5A) of the introduction tube (5) remains constant from the first end (10) to the second end (11).

15. Process according to claim 14 for the production of a product of particles having a cross-section perpendicular to their longitudinal axis, which in a first dimension is smaller than in a second dimension, **characterized in that** the particles are non-human mammalian animal sperms, comprising the step of detecting a property of the particles and the step of treating the particles in dependence on the detected property and/or the step of deflecting the particles in dependence on the detected property into separate containers for producing at least two different fractions.

## Revendications

1. Buse pour l'orientation de particules destinée à un cytomètre en flux présentant une première extrémité (10) et une deuxième extrémité (11) se trouvant à l'opposé ainsi qu'un petit tube d'alimentation (5) pour le liquide vecteur disposé avec son embout (12) dans le volume intérieur de la buse, ce petit tube étant disposé, dans une section voisine de son embout (12), sur un axe commun avec la buse, le petit tube d'alimentation (5) présentant une première ouverture d'admission (1) pour le liquide vecteur et son embout (12) étant distant d'une ouverture de sortie (7) disposée sur la deuxième extrémité (11) de la buse et la buse présentant une deuxième ouverture d'admission (4) pour le liquide de gaine, la section voisine de l'embout du petit tube d'alimentation (5) s'étirant sur toute la longueur du petit tube d'alimentation (5), disposée à l'intérieur de la buse, et la section intérieure (5A) du petit tube d'alimentation (5) pour le liquide vecteur étant perpendiculaire à l'axe longitudinal du petit tube d'alimentation (5), **caractérisée par le fait que** la section intérieure (5A) du petit tube d'alimentation (5), tout du moins dans la section voisine de son embout (12), est, dans une première dimension, au moins inférieure du facteur 0,3 à celle dans une deuxième dimension et que la section intérieure du petit tube d'alimentation (5) est rectangulaire ou elliptique et qu'elle est comprise entre 50 µm et 200 µm dans la première dimension et entre 75 µm et 300 µm dans la deuxième dimension et que la section intérieure (5A) du petit tube d'alimentation (5) reste constant de la première extrémité (10) à la deuxième extrémité (11).

2. Buse selon la revendication 1, **caractérisée par le fait que** le petit tube d'alimentation (5) présente une section intérieure rectangulaire (5A) dont l'étirement en longueur se trouve dans la deuxième dimension.

3. Buse selon la revendication 1, **caractérisée par le fait que** le petit tube d'alimentation (5) présente une section intérieure ovale (5A) dont l'étirement en longueur se trouve dans la deuxième dimension.

4. Buse selon l'une des revendications ci-avant, **caractérisée par le fait que** le volume intérieur de la buse est limité le long de son axe longitudinal à la section comprise entre l'ouverture de sortie (7) de la buse et l'embout (12) du petit tube d'alimentation (5).

5. Buse selon l'une des revendications ci-avant, **caractérisée par le fait qu'**un piézocristal (3) est disposé sur une paroi avec un écartement par rapport à la première extrémité (10) de la buse, paroi qui ferme la section de la buse jusqu'au niveau du petit tube d'alimentation (5).

6. Buse selon l'une des revendications ci-avant, **caractérisée par le fait que** la buse présente un raccord électrique qui contacte le volume intérieur de la buse.

7. Buse selon l'une des revendications ci-avant, **caractérisée par le fait que** le petit tube d'alimentation (5) à l'intérieur de la buse présente une épaisseur de paroi constante.

8. Buse selon l'une des revendications ci-avant, **caractérisée par le fait que** le petit tube d'alimentation (5) est formé par un support (13), un couvercle (16) distant du support (13) et deux blocs (14, 15) distants l'un de l'autre et disposés entre le support (13) et le couvercle (16).

9. Buse selon l'une des revendications ci-avant, **caractérisée par le fait que** le volume intérieur de la buse est limité par un support (13) et par un couvercle (16) distant de ce support et par des parois (17, 18) qui s'étirent à distance du tube d'alimentation entre le support (13) et le couvercle (16).

10. Buse selon la revendication 9, **caractérisée par le fait que** les parois (17, 18) sont disposées le long de l'axe longitudinal du petit tube (5) parallèlement aux surfaces des blocs (14, 15) qui sont dirigées vers les parois (17, 18).

11. Buse selon l'une des revendications 8 à 10, **caractérisée par le fait que** les surfaces se trouvant face à face du support (13) et du couvercle (16) sont parallèles et planes.

12. Buse selon l'une des revendications 8 à 11, **caractérisée par le fait que** les parois (17, 18), dans une section de la buse qui s'étend de l'embout (12) du petit tube d'alimentation (5) à l'ouverture de sortie (7), se rejoignent et forment entre elles l'ouverture de sortie (7).

13. Buse selon l'une des revendications 1 à 11, **caractérisée par le fait que** la buse se rétrécit dans une section (III) formée avec l'ouverture de sortie (7) disposée sur la deuxième extrémité (11) de la buse et qu'elle est formée par une garniture de buse (6) disposée de manière étanche au liquide.

14. Procédé pour l'orientation de particules qui, perpendiculairement à leur axe longitudinal, présentent une section qui, dans une première dimension, est inférieure à celle dans une deuxième dimension, dans un liquide vecteur à teneur en particules entouré de liquide gaine comportant les étapes : mise à disposition d'un liquide vecteur et d'un liquide gaine, pompage du liquide vecteur à travers une première ouverture d'admission (1) dans un petit tube d'alimentation (5) et pompage du liquide gaine à travers une deuxième ouverture d'admission (4) et écoulement d'un liquide vecteur entouré d'un liquide gaine sur la deuxième extrémité (11) d'une buse se trouvant face à la première extrémité (10) de la buse à travers une ouverture de sortie (7), le liquide vecteur étant guidé dans le petit tube d'alimentation (5) qui est disposé, dans une section voisine de son embout (12), sur un axe commun avec la buse et la section intérieure (5A) du petit tube d'alimentation (5) étant perpendiculaire à l'axe longitudinal du petit tube d'alimentation (5), la section voisine de l'embout du petit tube d'alimentation (5) s'étirant sur toute la longueur du petit tube d'alimentation (5), disposée à l'intérieur de la buse, **caractérisée par le fait que** la section intérieure (5A) du petit tube d'alimentation (5), tout du moins dans la section voisine de son embout (12), est, dans une première dimension, au moins inférieure du facteur 0,3 à celle dans une deuxième dimension et que la section intérieure du petit tube d'alimentation (5) est rectangulaire ou elliptique et qu'elle est comprise entre 50 µm et 200 µm dans la première dimension et entre 75 µm et 300 µm dans la deuxième dimension, et que la section intérieure (5A) du petit tube d'alimentation (5) reste constant de la première extrémité (10) à la deuxième extrémité (11).

15. Procédé selon la revendication 14 pour la fabrication d'une préparation de particules qui, perpendiculairement à leur axe longitudinal, présentent une section qui, dans une première dimension, est inférieure à celle dans une deuxième dimension, **caractérisé par le fait que** les particules sont des spermes de mammifères non humains, avec l'étape de la détection d'une propriété de particules et l'étape du traitement des particules en fonction de la propriété détectée et/ou l'étape de la déviation des particules en fonction de la propriété détectée dans des récipients séparés pour la production d'au moins deux fractions différentes.
